(19) Europäisches Patentamt
European Patent Office
Office européen des brevets

(11) Veröffentlichungsnummer: **0 130 140**
**B1**

(12) # EUROPÄISCHE PATENTSCHRIFT

(45) Veröffentlichungstag der Patentschrift: **23.01.91**

(21) Anmeldenummer: **84730069.6**

(22) Anmeldetag: **21.06.84**

(51) Int. Cl.[5]: **C 07 D 471/04,** A 61 K 31/435 // (C07D471/04, 221:00, 209:00)

(54) **Neue beta-Carboline, Verfahren zu ihrer Herstellung und ihre Verwendung als Arzneimittel (S).**

(30) Priorität: **23.06.83 DE 3322895**

(43) Veröffentlichungstag der Anmeldung:
**02.01.85 Patentblatt 85/01**

(45) Bekanntmachung des Hinweises auf die Patenterteilung:
**23.01.91 Patentblatt 91/04**

(84) Benannte Vertragsstaaten:
**AT BE CH DE FR GB IT LI LU NL SE**

(56) Entgegenhaltungen:
**EP-A-0 054 507**
**EP-A-0 110 813**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Seidelmann, Dieter, Dr.**
**Stierstrasse 14**
**D-1000 Berlin 41 (DE)**
Erfinder: **Schmiechen, Ralph, Dr.**
**Bayernring 27**
**D-1000 Berlin 42 (DE)**
Erfinder: **Huth, Andreas, Dr.**
**Kyllmannstrasse 15**
**D-1000 Berlin 39 (DE)**
Erfinder: **Rahtz, Dieter, Dr.**
**Krottnauer Strasse 24a**
**D-1000 Berlin 28 (DE)**
Erfinder: **Braestrup, Claus Thyco, Dr.**
**Frederiksborgvej 78**
**DK-4000 Roskilde (DK)**
Erfinder: **Engelstoft, Mogens, Dr.**
**Mosegaard Park 121**
**DK-3500 Vaerloese (DK)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 130 140 B1

**Beschreibung**

Die Erfindung betrifft neue substituierte β-Carboline, Verfahren zu ihrer Herstellung und ihre Verwendung als Arzneimittel.

Die erfindungsgemäßen Verbindungen besitzen wertvolle pharmakologische Eigenschaften. Sie beeinflussen insbesondere das Zentralnervensystem und eignen sich somit als Psychopharmaka, die im Vergleich mit den aus der EP—A—54507 bekannten im Benzyloxy-Rest unsubstituierten β-Carbolinen uberlegene Wirkung reigen.

Die erfindungsgemäß substituierten β-Carboline haben die allgemeine Formel I

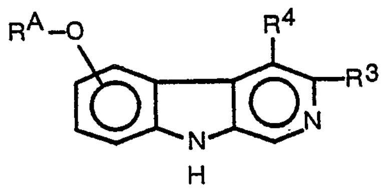

(I),

worin

$R^3$ den 5-Oxadiazolylrest

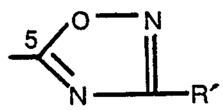

mit R′ in der Bedeutung von $C_{1-3}$-Alkyl und $C_{3-6}$-Cycloalkyl und den Alkyloxycarbonylrest —COOR″ mit R″ in der Bedeutung von $C_{1-4}$-Alkyl,

$R^4$ Wasserstoff, Methyl, Ethyl und Methoxymethyl und

$R^A$ den Arylrest

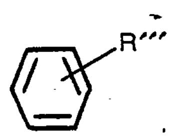

und den Aralkylrest

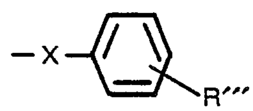

mit X in der Bedeutung von —$CH_2$—, das gegebenenfalls mit Methyl substituiert sein kann, und mit R‴ in der Bedeutung von Fluor, Chlor, Brom, $C_{1-3}$-Alkyl oder $C_{1-3}$-Alkoxy.

Die neuen β-Carboline der allgemeinen Formel I sind in 3-Stellung mit einem substituierten 5-Oxadiazolylrest oder mit einem Alkyloxycarbonylrest substituiert.

Unter Alkyl sind sowohl gerad- als auch verzweigtkettige Reste zu verstehen. Beispielsweise seien genannt Methyl, Ethyl, Propyl, iso-Propyl, n-Butyl, iso-Butyl und tert.-Butyl.

In 4-Stellung weisen die neuen β-Carboline entweder Wasserstoff oder niederes Alkyl wie Methyl oder Ethyl oder die Methoxymethylgruppe auf.

Der Substituent $R^A$, der über Sauerstoff am A-Ring gebunden ist, befindet sich vorzugsweise in 5- oder 6-Stellung.

Es ist bekannt, daß bestimmte Stellen im zentralen Nervensystem von Vertebraten eine hohe spezifische Affinität für die Bindung von 1.4- und 1.5-Benzodiazepinen aufweisen (Squires, R. F. und Braestrup, C., Nature (London) 266 (1977) 734). Die Stellen werden Benzodiazepin-Rezeptoren genannt.

Es wurde gefunden, daß die erfindungsgemäßen substituierten β-Carboline, obwohl sie sich in ihrer chemischen Struktur von den Benzodiazepinen stark unterscheiden, überraschenderweise eine starke Affinität und Spezifität für die Bindung an die Benzodiazepin-Rezeptoren zeigen, indem sie radioaktivmarkiertes Flunitrazepam von diesen Benzodiazepin-Rezeptoren verdrängen.

Die Verdrängungsaktivität der erfindungsgemäßen Verbindungen ist in der nachfolgenden Tabelle als $IC_{50}$- und $ED_{50}$-Wert angegeben. Der $IC_{50}$-Wert gibt die Konzentration an, die eine 50%ige Verdrängung der spezifischen Bindung von $^3H$-Flunitrazepam (1,0 nM, 0°C) in Proben mit einem Gesamtvolumen von 0,55 ml einer Suspension von Hirnmembran, z.B. von Ratten bewirkt.

Die Verdrängungsaktivität wird im in-vitro Test wie folgt bestimmt: 0,5 ml einer Suspension von unbehandeltem Ratten-Haupthirn in 25 mM $KH_2PO_4$, pH = 7,1 (5—10 mg Gewebe/Probe) wird für 40—60

Minuten bei 0°C zusammen mit $^3H$-Diazepam (spezifische Aktivität 14,4 Ci/mMol, 1,9 nM) oder $^3H$-Flunitrazepam (spezifische Aktivität 87 Ci/mMol, 1,0 nM) inkubiert. Nach Inkubation wird die Suspension durch eine Glasfritte filtriert, der Rückstand 2 mal mit kalter Pufferlösung gewaschen und die Radioaktivität am Scintillationszähler gemessen.

Der Versuch wird dann wiederholt, jedoch so, daß vor der Zugabe des radioaktiv-markiertem Benzodiazepins eine bestimmte Menge oder eine überschüssige Menge der Verbindüng, deren Verdrängungsaktivität zu bestimmen ist, zugesetzt wird. Auf Grundlage der erhaltenen Werte wird der $IC_{50}$-Wert berechnet.

Der $ED_{50}$-Wert stellt die Dosis einer Versuchssubstanz dar, die einer Reduktion der spezifischen Bindung des Flunitrazepams an dem Benzodiazepin-Rezeptor in einem lebenden Gehirn auf 50% des Kontrollwertes bewirkt.

Der in-vivo Test wird wie folgt ausgeführt:

Gruppen von Mäusen wird die Versuchssubstanz bei unterschiedlichen Dosen und normalerweise subcutan injiziert. Nach 15 Minuten wird den Mäusen das $^3H$-Flunitrazepam intravenös verabreicht. Nach weiteren 20 Minuten werden die Mäuse getötet, ihre Vorderhirnhäute entfernt und die Radioaktivität der Vorderhirnhäute durch Scintillationszählung gemessen. Der $ED_{50}$-Wert wird mit Hilfe der Dosis/Wirkungs-Kurven bestimmt.

Die erfindungsgemäßen Verbindungen zeigen im pharmakoligischen Test anxiolytische, antiaggressiv und anti-konvulsive Wirksamkeit. Zur Untersuchung der anti-konvulsiven Wirkung wurden zwei Testanordnungen eingesetzt. Einmal wurde die Aufhebung der mit Pentylentetrazol (Pentazol) und zum anderen der mit 6.7-Dimethoxy-4-ethyl-β-carbolin-3-carbonsäuremethylester (DMCM) induzierten Krämpfe untersucht. Pentazol bzw. DMCM werden in einer Menge von 15 mg/kg als wässrige Lösung (pH 7) intraperitoneal bzw. 150 mg/kg als salzsaure Lösung (pH 2—3) subcutan 15—30 Minuten nach der intraperitonealen Applikation der Testsubstanz gegeben. Diese Mengen induzieren klonische und tonische Krämpfe, die bei unbehandelten Tieren zum Tode führen. Die Zahl der Mäuse, die Krämpfe zeigen, und die Zahl derer, die 30 Minuten nach der Pentazol- bzw. DMCM-Applikation gestorben sind, wird registriert.

Die in der Tabelle angegebenen $ED_{50}$-Werte wurden nach der Methode von Litchfield und Wilcoxon (J. Pharmacol. exp. Ther. *96* (1949) 99—103) als die Menge der antagonistisch wirkenden Substanz bestimmt, die 50% der Tiere von Krämpfen und Tod schützt.

Des weiteren zeigen die erfindungsgemäßen Verbindungen eine krampf-lösende bzw. krampaufhebende Wirkung im Audiogenic Seizure Test. Hierzu wird 18—21 Tage alten männlichen Mäusen mit einem Gewicht von 8—12 g (DBA/2-Mäuse) 30 Minuten von dem Test die zu untersuchende Substanz als Ultraschall-Mikrosuspension in Wasser/Cremofor® EL (95:5) intraperitoneal verabreicht. Anschließend werden die Tiere in einem schalldichten Holzkasten (25 × 22 × 15 cm) einem Sinus-Ton von 14 kHz bei 111 dB ausgesetzt. Der Ton wird sofort nach dem Überführen des Tieres in den Kasten erzeugt. Das Auftreten der klonischen Krämpfe wird für 30 sec registriert. Der $ED_{50}$-Wert in der Tabelle gibt an, bei welcher Dosis 40% der Mäuse bei 111 dB (80% der Mäuse zeigen Krämpfe bei 111 dB) keine Krämpfe zeigen.

TABELLE

| Substituent R''' | $IC_{50}$ ng/ml (in vitro) | $ED_{50}$ mg/kg (in vivo) | $ED_{50}$ mg/kg | | |
|---|---|---|---|---|---|
| | | | Pentazol | DMCM | 111 dB |
| 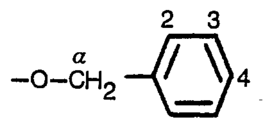 | 2 | 4 | 15 | 0,6 | 0,5 |
| 2-flour | 0,3 | 1,1 | 13 | 0,4 | 0,05 |
| 3-fluor | 0,3 | 2,1 | 12 | 0,3 | 0,05 |
| 4-fluor | 0,4 | 0,5 | 30 | 0,1 | 0,2 |
| 3-chlor | 1,4 | 2,8 | 0,9 | 0,3 | 0,08 |
| 4-chlor | 0,9 | 5,6 | >30 | | |
| 3-brom | 0,8 | >9 | 6 | | |
| 3-methoxy | 0,5 | 3,7 | >100 | | |
| 4-methyl | 0,9 | 10 | 30 | | |
| α-methyl, 4-chlor | 1,1 | 0,5 | 3 | 0,1 | 0,01 |
| α-methyl, 3-chlor | 4,3 | 1,3 | 2 | | |

Pharmakologische Eigenschaften von R'''-substituierten 5-Benzyloxy-4-methoxymethyl-β-carbolin-3-carbonsäureethylestern
DMCM = 6.7-Dimethoxy-4-ethyl-β-carbolin-3-carbonsäuremethylester.

Die erfindungsgemäßen Verbindungen erscheinen aufgrund ihrer biologischen Wirksamkeit als Psychopharmaka für die Humanmedizin geeignet. Sie können zu psychopharmazeutischen Präparationen, zum Beispiel für die orale und parenterale Anwendung, formuliert angewendet werden.

Als Formulierungshilfsstoffe sind physiologisch verträgliche organische und anorganische Trägersubstanzen geeignet, die gegenüber den erfindungsgemäßen Verbindungen inert sind.

Als Trägersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäuremono- und diglyceride, Pentaerythritolfettsäureester, Hydroxymethylcellulose und Polyvinylpyrrolidon.

Die pharmazeutischen Präparationen können sterilisiert und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Für die orale Anwendung sind insbesondere Tabletten, Dragees oder Kapseln mit Talkum und/oder einem Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt wird.

Die erfindungsgemäßen Verbindungen werden in einer Dosiseinheit von 0,05 bis 10 mg aktiver Substanz in einem physiologisch verträglichen Träger eingebracht.

Die erfindungsgemäßen Verbindungen werden in einer Dosis von 0,1 bis 300 mg/Tag, vorzugsweise 1—30 mg/Tag, angewendet.

Die Herstellung der erfindungsgemäßen Verbindungen der allgemeinen Formel I erfolgt nach an sich bekannten Methoden, dadurch, daß man ein substituiertes Hydroxy-β-carbolin der allgemeinen Formel II

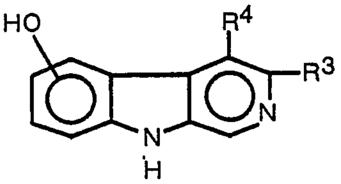

(II),

worin $R^3$ und $R^4$ die oben angegebene Bedeutung haben, nach an sich bekannten Methoden mit einem reaktionsfähigen Derivat der allgemeinen Formel $R^AY$ umsetzt, wobei Y für Chlor, Brom, Jod oder den Tosylrest steht und $R^A$ die oben angegebene Bedeutung hat und gewünschtenfalls die so erhaltene Verbindung der Formel I, in welcher R'' für Methyl oder Ethyl steht, in an sich bekannter Weise umestert oder eine substituierte β-Carbolin-3-carbonsäure der allgemeinen Formel III

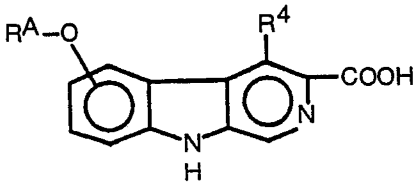

(III)

worin $R^A$ und $R^4$ die oben angegebene Bedeutung haben, in an sich bekannter Weise mit einem Amidoxim der allgemeinen Formel $R'—C(=NOH)NH_2$, worin R' die oben angegebene Bedeutung hat, zum 5-Oxadiazolyl-Derivat umsetzt.

Die Verätherung des im A-Ring hydroxy β-Carbolinderivats der Formel II erfolgt durch Umsetzung mit einer reaktionsfähigen Aryl- oder Aralkylverbindung in einem polaren Lösungsmittel wie Ethanol, Acetonitril oder Dimethylformamid in Gegenwärt eines Alkalimetallcarbonats wie z.B. Kaliumcarbonat oder Basen wie z.B. Kaliumhydroxid, 1.5-Diazabicyclo(5.4.0)undec-5-en (DBU) und 1.4-Diazabicyclo(2.2.2)octan (Dabco). Als reaktionsfähige Aryl- oder Aralkylverbindung sind insbesondere die Halogenide wie Chlorid, Bromid und Jodid sowie Mesylate und Tosylate geeignet.

Zur Veresterung wird die freie β-Carbolin-3-carbonsäure z.B. mit Cäsiumcarbonat in das Cäsiumsalz überführt und anschließend mit dem entsprechenden Alkylhalogenid zur Reaktion gebracht.

Zur Umesterung wird der vorliegende Ester der Formel I mit einem Alkohol R''OH in Gegenwart katalytischer Mengen von R''ONa oder NaH 3—6 Stunden auf Temperaturen zwischen 60 und 120°C erhitzt. Gegebenenfalls kann die Umesterung auch mit dem Alkohol R''OH in Gegenwart eines sauren Katalysators wie p-Toluolsulfonsäure, Salzsäure oder Kupfer-II-chlorid vorgenommen werden.

Stellt der Substituent $R^3$ einen 5-Oxadiazolylrest dar, so wird die freie β-Carbolin-3-carbonsäure der Formel III mit einem Amidoxim der Formel $R'—C(=NOH)NH_2$ in einem Lösungsmittel, das über 100°C siedet und gegenüber den Reaktanten inert ist, bei der Rückflußtemperatur des Reaktionsgemisches zur Kondensation gebracht. Geeignete Lösungsmittel für die Kondensationsreaktion sind beispielsweise Toluol und Dimethylformamid.

Zweckmäßigerweise wird die freie β-Carbolin-3-carbonsäure der Formel III vor der Kondensationsreaktion in geeigneter Weise aktiviert. Möglich ist die Überführung der Säure in das gemischte Anhydrid, in den aktivierten Ester oder in das Chlorid. Gut bewährt hat sich eine Aktivierung mit Imidazol/Thionylchlorid in einem aprotischen Lösungsmittel wie Dioxan, Tetrahydrofuran, Dimethylformamid oder N-Methylpyrrolidon bei Temperaturen zwischen 0 und 50°C, vorzugsweise Raumtemperatur.

Herstellung des Ausgangsmaterials

Das Ausgangsmaterial kann nach verschiedenen Methoden hergestellt werden.

Methode a)

10 g 5-Benzyloxy-4-methoxymethyl-β-carbolin-3-carbonsäureethylester werden in 100 ml Ethanol mit 4 g Raney-Nickel 3 Stunden am Rückfluß gekocht. Nach dem Abfiltrieren des Katalysators wird das Filtrat im Vakuum eingeengt. Der Rückstand wird über Kieselgel mit Methylenchlorid + Ethanol/1000 + 75 chromatographiert. Man erhält 7,2 g 5-Hydroxy-4-methoxymethyl-β-carbolin-3-carbonsäureethylester vom Schmelzpunkt 179—181°C.

Methode b)

21 g 5-Benzyloxy-4-methoxymethyl-β-carbolin-3-carbonsäureethylester werden in 250 ml Ethanol mit 10 g Palladium/Kohle (10 %ig), 3,5 Stunden bei Raumtemperatur unter Wasserstoffnormaldruck hydriert. Nach Filtrieren und Einengen wird der Rückstand wie bei Methode a) chromatographiert. Man erhält 15,1 g 5-Hydroxy-4-methoxymethyl-β-carbolin-3-carbonsäureethylester vom Schmelzpunkt 179—181°C.

Methode c)

2,19 g 5-Benzyloxy-4-methoxymethyl-3-ethoxycarbonyl-1.2.3.4-tetrahydro-β-carbolin-1-carbonsäure

werden mit 0,2 g Palladium/Kohle (10 %ig), in 100 ml Xylol 3 Stunden am Rückfluß erhitzt. Nach Abfiltrieren des Katalysators wird das Filtrat eingeengt. Der Rückstnad wird über Kieselgel chromatographiert (Methylenchlorid + Ethanol/10 + 1). Man erhält 0,57 g 5-Hydroxy-4-methoxymethyl-β-carbolin-3-carbonsäureethylester vom Schmelzpunkt 179—181°C.

Das für diese Methode verwendete Ausgangsmaterial kann wie folgt hergestellt werden: 37,6 g 3-(4-Benzyloxyindol-3-yl)-2-amino-5-oxa-hexansäureethylester werden in 200 ml Essigester gelöst. Dazu wird unter kräftigem Rühren eine Lösung aus 10,8 g Glyoxylsäure-Monohydrat in 120 ml Wasser getropft. Das Reaktionsgemisch wird anschließend mit 10 %iger Kaliumcarbonatlösung auf pH 4 eingestellt und 14 Stunden bei Raumtemperatur gerührt. Das ausgefallene gelbe Kristallisat wird abgesaugt, mit Essigester gewaschen und getrocknet. Man erhält 20,3 g 5-Benzyloxy-4-methoxymethyl-3-ethoxy-carbonyl-1.2.3.4-tetrahydro-β-carbolin-1-carbonsäure als hellgelbe Kristalle vom Schmelzpunkt 139—142°C.

Nach dem in Klammern angegebenen Methoden a), b) und c) werden die folgenden Ausgangsmaterialien hergestellt:

6-Hydroxy-4-methyl-β-carbolin-3-carbonsäureethylester, F. 269—270°C, (b + c);
6-Hydroxy-4-ethyl-β-carbolin-3-carbonsäureethylester, F. 260—263°C, (b);
6-Hydroxy-β-carbolin-3-carbonsäureethylester, F. 248—250°C, (Zersetzung), (b);
6-Hydroxy-4-methoxymethyl-β-carbolin-3-carbonsäureethylester, F. 164—166°C, (b + c);
5-Hydroxy-β-carbolin-3-carbonsäureethylester, F. 255°C (Zersetzung), (b);
5-Hydroxy-4-methyl-β-carbolin-3-carbonsäureethylester, F. 240—243°C (Zersetzung), (a + c);
5-Hydroxy-4-ethyl-β-carbolin-3-carbonsäureethylester, F. 188—190°C, (a).

Die nachfolgenden Beispiele sollen das erfindungsgemäße Verfahren erläutern.

Beispiel 1

0,3 g 5-Hydroxy-4-methoxymethyl-β-carbolin-3-carbonsäureethylester werden in 60 ml Ethanol mit 0,3 g Kaliumcarbonat und 0,174 g 3-Fluorbenzylchlorid unter Stickstoff-Atmosphäre 4 Stunden am RÜckfluß gekocht. Nach Filtrieren und Einengen im Vakuum wird der Rückstand über Kieselgel chromatographiert (Methylenchlorid + Ethanol/1000 + 25). Man erhält 0,167 g 5-(3-Fluorbenzyloxy)-4-methoxymethyl-β-carbolin-3-carbolin-3-carbonsäureethylester vom Schmelzpunkt 188,0°C.

Beispiel 2

Analog Beispiel 1 werden aus dem 5-Hydroxy-4-methoxymethyl-β-carbolin-3-carbonsäureethylester und dem entsprechenden substituierten Benzylhalogenid die folgenden Verbindungen hergestellt;

5-(2-Fluorbenzyloxy)-4-methoxymethyl-β-carbolin-3-carbonsäureethylester, F. 139—140°C;
5-(4-Fluorbenzyloxy)-4-methoxymethyl-β-carbolin-3-carbonsäureethylester, F. 174—176°C;
5-(4-Chlorbenzyloxy)-4-methoxymethyl-β-carbolin-3-carbonsäureethylester, F. 191—193°C;
5-(3-Chlorbenzyloxy)-4-methoxymethyl-β-carbolin-3-carbonsäureethylester, F. 196°C;
5-(2-Chlorbenzyloxy)-4-methoxymethyl-β-carbolin-3-carbonsäureethylester, F. 174—176°C;
5-(3-Brombenzyloxy)-4-methoxymethyl-β-carbolin-3-carbonsäureethylester, F. 209°C;
5-(3-Methoxybenzyloxy)-4-methoxymethyl-β-carbolin-3-carbonsäureethylester, F. 190°C;
5-(4-Methylbenzyloxy)-4-methoxymethyl-β-carbolin-3-carbonsäureethylester, F. 164°C;
5-[1-(3-Chlorphenyl)-ethoxy]-4-methoxymethyl-β-carbolin-3-carbonsäureethylester, F. 166°C und
5-[1-(4-Chlorphenyl)-ethoxy]-4-methoxymethyl-β-carbolin-3-carbonsäureethylester, F. 158°C.

Beispiel 3

Analog Beispiel 1 werden aus 5-Hydroxy-4-methyl-β-carbolin-3-carbonsäureethylester und dem entsprechenden substituierten Benzylhalogenid die folgenden Verbindungen hergestellt:

5-(2-Chlorbenzyloxy)-4-methyl-β-carbolin-3-carbonsäureethylester und
5-(3-Methoxybenzyloxy)-4-methyl-β-carbolin-3-carbonsäureethylester.

Beispiel 4

Analog Beispiel 1 werden aus 5-Hydroxy-4-ethyl-β-carbolin-3-carbonsäureethylester und dem entsprechenden substituierten Benzylhalogenid die folgenden Verbindungen hergestellt:

5-(3-Chlorbenzyloxy)-4-ethyl-β-carbolin-3-carbonsäureethylester;
5-(3-Fluorbenzyloxy)-4-ethyl-β-carbolin-3-carbonsäureethylester und
5-[1-(3-Chlorphenyl)-ethoxy]-4-ethyl-β-carbolin-3-carbonsäureethylester.

Beispiel 5

Analog Beispiel 1 werden aus 5-Hydroxy-β-carbolin-3-carbonsäureethylester und dem entsprechenden substituierten Benzylhalogenid die folgenden Verbindungen hergestellt:

5-(3-Chlorbenzyloxy)-β-carbolin-3-carbonsäureethylester und
5-(3-Fluorbenzyloxy)-β-carbolin-3-carbonsäureethylester.

Beispiel 6

Analog Beispiel 1 werden aus 6-Hydroxy-β-carbolin-3-carbonsäureethylester und dem entsprechenden substituierten Benzylhalogenid die folgenden Verbindungen hergestellt:

6-(3-Chlorbenzyloxy)-β-carbolin-3-carbonsäureethylester und
6-(4-Fluorbenzyloxy)-β-carbolin-3-carbonsäureethylester.

Beispiel 7

Analog Beispiel 1 werden aus 6-Hydroxy-4-methoxymethyl-β-carbolin-3-carbonsäureethylester und dem entsprechenden substituierten Benzylhalogenid die folgenden Verbindungen hergestellt:

6-(3-Chlorbenzyloxy)-4-methoxymethyl-β-carbolin-3-carbonsäureethylester, F. 185—187°C;
6-(4-Methylbenzyloxy)-4-methoxymethyl-β-carbolin-3-carbonsäureethylester, F. 161 und
6-[1-(3-Chlorphenyl)-ethoxy]-4-methoxymethyl-β-carbolin-3-carbonsäureethylester, F. 119°C.

Beispiel 8

Analog Beispiel 1 werden aus 6-Hydroxy-4-methyl-β-carbolin-3-carbonsäureethylester und dem entsprechenden substituierten Benzylhalogenid die folgenden Verbindungen hergestellt:

6-(3-Chlorbenzyloxy)-4-methyl-β-carbolin-3-carbonsäureethylester und
6-(3-Fluorbenzyloxy)-4-methyl-β-carbolin-3-carbonsäureethylester.

Beispiel 9

Methode A

1 g des im A-Ring substituierten β-Carbolin-3-carbonsäureethylesters wird in 10 ml des gewünschten Alkohols suspendiert bzw. gelöst und nach Zugabe von 20 mg Kupfer-II-bromid 5—24 Stunden auf 50°C erwärmt. Die Reaktionszeit wird durch Dünnschichtchromatographie ermittelt. Das Reaktionsgemisch wird in Eis/Wasser eingerührt und das aufgefallene Produkt abfiltriert, mit Wasser gewaschen, getrocknet und anschließend umkristallisiert.

Methode B

In 10 ml des gewünschten Alkohols werden 100 mg Natriummetall gelöst, dann 1 g des oben verwendeten Ethylesterderivats hinzugefügt, das Reaktionsgemisch auf 60—80°C erwärmt und nach Beendigung der Reaktion wie bei Methode A aufgearbeitet.

Es werden die folgenden Verbindungen nach Methode A hergestellt:

5-(4-Chlorbenzyloxy)-4-methoxymethyl-β-carbolin-3-carbonsäure-n-propylester;
5-[1-(4-Chlorphenyl)-ethoxy]-4-methoxymethyl-β-carbolin-3-carbonsäure-isopropylester und
6-(3-Fluorbenzyloxy)-4-ethyl-β-carbolin-3-carbonsäure-n-butylester.

Nach Methode B werden die folgenden Verbindungen hergestellt:

6-(4-Chlorbenzyloxy)-4-ethyl-β-carbolin-3-carbonsäuremethylester und
6-(4-Chlorbenzyloxy)-β-carbolin-3-carbonsäure-methylester.

Beispiel 10

Es werden 0,2 g 4-Methoxybrombenzol, 0,3 g 6-Hydroxy-4-methoxymethyl-β-carbolin-3-carbonsäure-ethylester und 0,15 g Kupfer-I-oxid 35 Stunden in 5 ml Collidin zum Sieden erhitzt. Nach dem Abkühlen wird filtriert und im Vakuum eingedampft. Der Rückstand wird in Essigester aufgenommen, mehrfach mit eiskalter 25 %iger Ammoniaklösung extrahiert und dann mit gesättigter Kochsalzlösung gewaschen, über Calciumsulfat getrocknet und eingedampft. Durch Chromatographie an Kieselgel mit Methylenchlorid-Ethanol (10:1) erhält man 0,17 g 6-(4-Methoxy-phenoxy)-4-methoxymethyl-β-carbonsäureethylester vom Schmelzpunkt 154,0°C.

Beispiel 11

1,36 g Imidazol werden in 15 ml absolutem Tetrahydrofuran mit 0,36 ml Thionylchlorid in 5 ml absolutem Tetrahydrofuran versetzt. Nach 15 Minuten Rühren bei Raumtemperatur wird vom Niederschlag abgesaugt. Das Filtrat wird zu einer Suspension von 2,55 g 5-(3-Chlorbenzyloxy)-4-methoxymethyl-β-carbolin-3-carbonsäure in 50 ml absolutem Dimethylformamid gegeben. Nach 1 Stunde Rühren bei Raumtemperatur versetzt man mit 180 µl Wasser und anschließend mit 2,6 g Propionamidoxim, destilliert das Tetrahydrofuran ab und erhitzt die Reaktionslösung 3 Stunden am Rückfluß. Nach Abdestillieren des Lösungsmittels verteilt man in Methylenchlorid/gesättiger Natriumbicarbonatlösung, wäscht die organische Phase mit gesättigter Natriumchlorid-Lösung neutral, trocknet über Magnesiumsulfat und destilliert das Lösungsmittel im Vakuum ab. Das Reaktionsprodukt wird aus 2-Propanol umkristallisiert. Man erhält 1,3 g 5-(3-Chlorbenzyloxy)-3-(5′-(3′-ethyl-(1.2.4-oxadiazol))-yl)-4-methoxymethyl-β-carbolin vom Schmelzpunkt 182—187°C.

Das als Ausgangsmaterial verwendete 5-(3-Chlorbenzyloxy)-4-methoxymethyl-β-carbolin-3-carbonsäure erhält man nach Verseifung des entsprechenden Ethylesters.

Beispiel 12

Nach der im Beispiel 11 beschriebenen Methode werden aus der entsprechenden substituierten β-Carbolin-3-carbonsäure die folgenden Verbindungen hergestellt:

5-[1-(3-Chlorphenyl)-ethoxy]-3-(5'-(3'-ethyl-(1.2.4-oxadiazol))-yl)-4-methoxymethyl-β-carbolin, F. 179—181°C;

6-(3-Chlorbenzyloxy)-3-(5'-(3'-ethyl-(1.2.4-oxadiazol))-yl)-4-methoxymethyl-β-carbolin, F. 199—202°C und

6-[1-(3-Chlorphenyl)-ethoxy]-3-(5'-(3'-ethyl-(1.2.4-oxadiazol))-yl)-4-methoxymethyl-β-carbolin, F. 153—157°C.

**Patentansprüche**

1. Verfahren zur Herstellung von neuen substituierten β-Carbolinen der allgemeinen Formel I

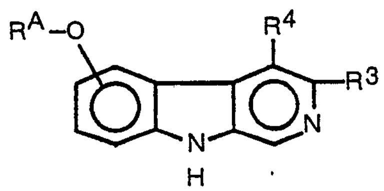

(I)

worin

$R^3$ den 5-Oxadiazolylrest

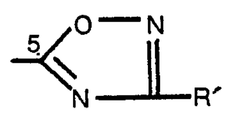

mit R' in der Bedeutung von $C_{1-3}$-Alkyl und $C_{3-6}$-Cycloalkyl und den Alkyloxycarbonlyrest —COOR'' mit R'' in der Bedeutung von $C_{1-4}$-Alkyl,

$R^4$ Wasserstoff, Methyl, Ethyl und Methoxymethyl und

$R^A$ den Arylrest

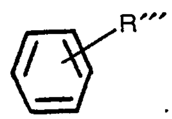

und den Aralkylrest

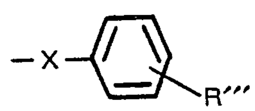

mit X in der Bedeutung von —$CH_2$—, das gegebenenfalls mit Methyl substituiert sein kann, und mit R''' in der Bedeutung von Fluor, Chlor, Brom, $C_{1-3}$-Alkyl oder $C_{1-3}$-Alkoxy, dadurch gekennzeichnet, daß man ein substituiertes Hydroxy-β-carbolin der allgemeinen Formel II

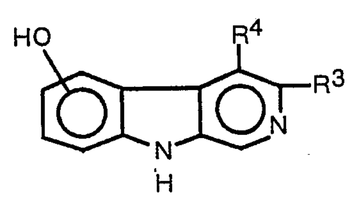

(II)

worin $R^3$ und $R^4$ die oben angegebene Bedeutung haben, nach an sich bekannten Methoden mit einem reaktionsfähigen Derivat der allgemeinen Formel $R^AY$ umsetzt, wobei Y für Chlor, Brom, Jod oder den Tosylrest steht und $R^A$ die oben angegebene Bedeutung hat und gewünschtenfalls die so erhaltene Verbindung der Formel I, in welcher R'' für Methyl oder Ethyl steht, in an sich bekannter Weise umestert oder eine substituierte β-Carbolin-3-carbonsäure der allgemeinen Formel III

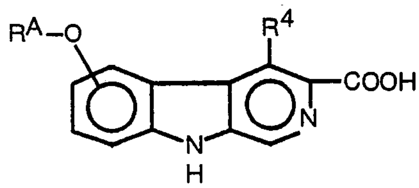

(III)

worin $R^A$ und $R^4$ die oben angegebene Bedeutung haben, in an sich bekannter Weise mit einem Amidoxim der allgemeinen Formel $R'—C(=NOH)NH_2$, worin R′ die oben angegebene Bedeutung hat, zum 5-Oxadiazolyl-Derivat umsetzt.

2. Substituierte β-Carboline der allgemeinen Formel I

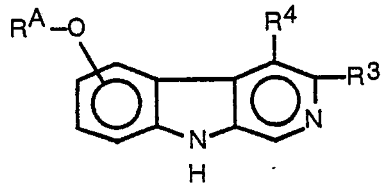

(I)

worin

$R^3$ den 5-Oxadiazolylrest

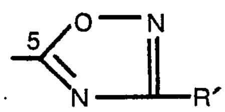

mit R′ in der Bedeutung von $C_{1-3}$-Alkyl und $C_{3-6}$-Cycloalkyl und den Alkyloxycarbonylrest —COOR″ mit R″ in der Bedeutung von $C_{1-4}$-Alkyl,

$R^4$ Wasserstoff, Methyl, Ethyl und Methoxymethyl und

$R^A$ den Arylrest

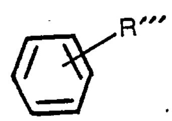

und den Aralkylrest

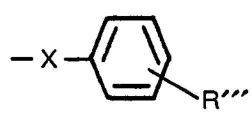

mit X in der Bedeutung von $—CH_2—$, das gegebenenfalls mit Methyl substituiert sein kann, und mit R‴ in der Bedeutung von Fluor, Chlor, Brom, $C_{1-3}$-Alkyl oder $C_{1-3}$-Alkoxy.

3. 5-(3-Chlorbenzyloxy)-3-(5′-(3′-ethyl-(1.2.4-oxadiazol)-yl)-4-methoxymethyl-β-carbolin.

4. 5-(3-Chlorbenzyloxy)-4-methoxymethyl-β-carbolin-3-carbonsäureethylester.

5. Arzneimittel auf Basis der Verbindungen gemäß den Ansprüchen 2—4.

**Revendications**

1. Procédé pour préparer de nouvelles β-carbolines substituées qui répondent à la formule générale I

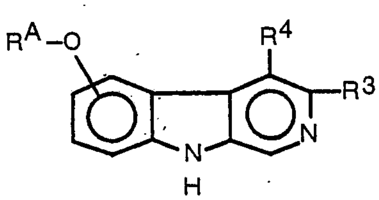

(I)

dans laquelle

$R^3$ représente;

un radical oxadiazolyle-5

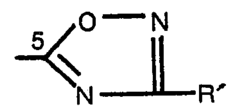

dans lequel R′ désigne un alkyle en $C_1$—$C_3$ ou un cycloalkyle en $C_3$—$C_6$, ou un radical alcoxycarbonyle —COOR″ dans lequel R″ rerpésente un alkyle en $C_1$—$C_4$,

$R^4$ représente l'hydrogène ou un radical méthyle, éthyle ou méthoxyméthyle, et

$R^A$ représente un radical aryle

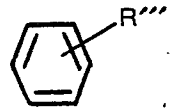

ou un radical aralkyle

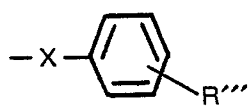

dans lesquels X représente un radical —$CH_2$— éventuellement porteur d'un méthyle, et R‴ représente le fluor, le chlore, le brome, un alkyle en $C_1$—$C_3$ ou un alcoxy en $C_1$—$C_3$, procédé caractérisé en ce qu'on fait réagir une hydroxy-β-carboline substituée répondant à la formule II

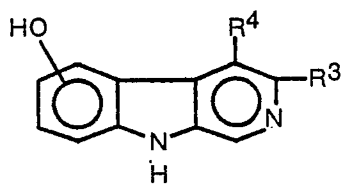

(II)

dans laquelle $R^3$ et $R^4$ ont les significations qui leur ont été données ci-dessus, avec un dérivé réactif répondant à la formule générale $R^AY$ dans laquelle Y représente le chlore, le brome, l'iode ou un radical tosyle et $R^A$ a la signification qui lui a été donnée plus huat, et, si on le désiré, on transestérifie de manière connue le composé de formule I ainsi obtenu dans lequel R″ représente un radical méthyle ou éthyle, ou un fait réagir de manière connue un acide β-carboline-carboxylique-3-substitué répondant à la formule générale III:

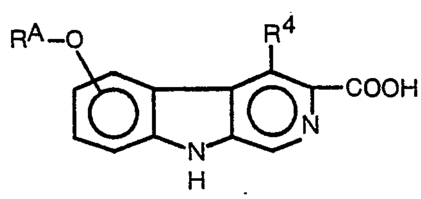

(III)

dans laquelle $R^A$ et $R^4$ ont les significations qui leur ont été données ci-dessus, avec une amide-oxime répondant à la formule générale: R′—C(=NOH)$H_2$ dans laquelle R′ a la signification qui lui a été donnée plus haut, de manière à obtenir le dérivé oxadiazolylique-5.

2. β-Carbolines substituées répondant à la formule générale I

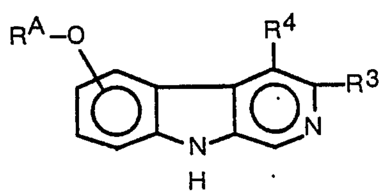

(I)

dans laquelle

$R^3$ représente;

un radical oxadiazolyle-5

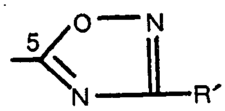

dans lequel R′ désigne un alkyle en $C_1$—$C_3$ ou un cycloalkyle en $C_3$—$C_6$, ou un radical alcoxycarbonyle —COOR″ dans lequel R″ représente un alkyle en $C_1$—$C_4$,

$R^4$ représente l'hydrogène ou un radical méthyle, éthyle ou méthoxyméthyle, et

$R^A$ représente un radical aryle

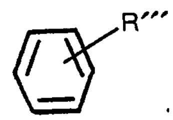

ou un radical aralkyle

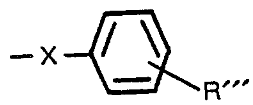

dans lesquels X représente un radical —$CH_2$— éventuellement porteur d'un méthyle, et R‴ représente le fluor, le chlore, le brome, un alkyle en $C_1$—$C_3$ ou un alcoxy en $C_1$—$C_3$,

3. (Chloro-3 benzyloxy)-5 (éthyl-3 oxadiazole-1,2,4 yl-5)-3 méthoxyméthyl-4 β-carboline.

4. (Chloro-3 benzyloxy)-5 méthoxyméthyl-4 β-carboline-carboxylate-3 d'éthyle.

5. Médicament à base des composés selon l'une quelconque des revendications 2 à 4.

**Claims**

1. Process for the manufacture of novel substituted β-carbolines of the general formula I

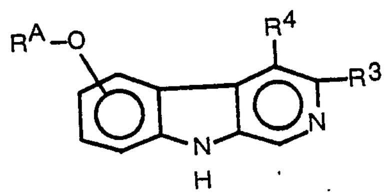

(I)

in which

$R^3$ represents a 5-oxadiazolyl radical

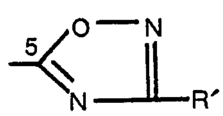

wherein R′ represents $C_{1-3}$-alkyl or $C_{3-6}$-cycloalkyl or an alkoxycarbonyl radical —COOR″ wherein R″ represents $C_{1-4}$-alkyl,

$R^4$ represents hydrogen, methyl, ethyl or methoxymethyl, and

$R^A$ represents an aryl radical

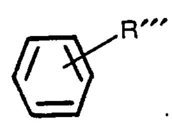

or an aralkyl radical

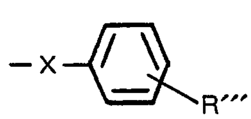

wherein X represents —$CH_2$—, which may optionally be substituted by methyl, and R‴ represents fluorine,

chlorine, bromine, $C_{1-3}$-alkyl or $C_{1-3}$-alkoxy, characterised in that a substituted hydroxy-β-carboline of the general formula II

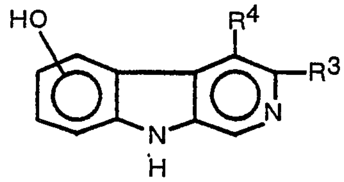

(II)

in which $R^3$ und $R^4$ have the meanings given above, is reacted according to methods known *per se* with a reactive derivative of the general formula $R^AY$ in which Y represents chlorine, bromine, iodine or a tosyl radical and $R^A$ has the meaning given above and, if desired, the compound of formula I so obtained in which R″ represents methyl or ethyl is transesterified in a manner known *per se*, or a substituted β-carboline-3-carboxylic acid of the general formula III

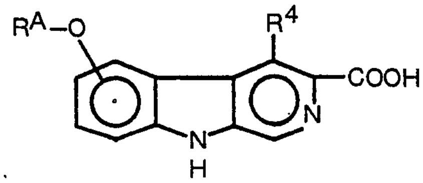

(III)

in which $R^A$ and $R^4$ have the meanings given above, is reacted in a manner known *per se* with an amide oxime of the general formula R′—C(=NOH)$NH_2$, wherein R′ has the meaning given above, to form the 5-oxadiazolyl derivative.

2. Substituted β-carbolines of the general formula I

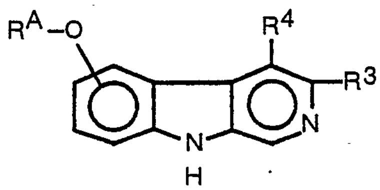

(I)

in which

$R^3$ represents a 5-oxadiazolyl radical

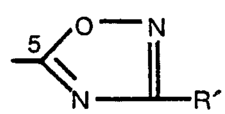

wherein R′ represents $C_{1-3}$-alkyl or $C_{3-6}$-cycloalkyl or an alkoxycarbonyl radical —COOR″ wherein R″ represents $C_{1-4}$-alkyl,

$R^4$ represents hydrogen, methyl, ethyl or methoxymethyl, and

$R^A$ represents an aryl radical

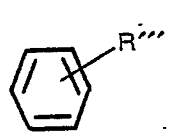

or an aralkyl radical

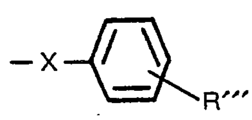

wherein X represents —$CH_2$—, which may optionally be substituted by methyl, and R‴ represents fluorine, chlorine, bromine, $C_{1-3}$-alkyl or $C_{1-3}$-alkoxy.

3. 5-(3-Chlorobenzyloxy)-3-(5′-(3′-ethyl-(1,2,4-oxadiazol))-yl)-4-methoxymethyl-β-carboline.

4. 5-(3-Chlorobenzyloxy)-4-methoxymethyl-β-carboline-3-carboxylic acid ethyl ester.

5. Medicament based on the compounds according to claims 2 to 4.